# EUROPÄISCHE PATENTSCHRIFT

(11) **EP 1 633 410 B1**
(45) Veröffentlichungstag und Bekanntmachung des Hinweises auf die Patenterteilung: **17.05.2017**
(21) Anmeldenummer: 04739764.1
(22) Anmeldetag: 09.06.2004
(51) Int. Cl.: A61L 31/10, A61F 2/82, A61L 31/14

(54) **BIOABBAUBARE STENTS**
BIODEGRADABLE STENTS
ENDOPROTHESES BIODEGRADABLES

(30) Priorität: 13.06.2003 DE 10326782; 13.06.2003 DE 10326778; 10.12.2003 DE 10357747; 10.12.2003 DE 10357744
(43) Veröffentlichungstag der Anmeldung: 15.03.2006
(73) Patentinhaber: Helmholtz-Zentrum Geesthacht Zentrum für Material- und Küstenforschung GmbH, 21502 Geesthacht (DE)
(72) Erfinder: SIMON, Peter, 52068 Aachen (DE); LENDLEIN, Andreas, 14167 Berlin (DE); SCHNITTER, Birgit, 52531 Übach-Palenberg (DE)
(74) Vertreter: Gulde & Partner
(86) Internationale Anmeldenummer: PCT/EP2004/006261
(87) Internationale Veröffentlichungsnummer: WO 2004/110515

(56) Entgegenhaltungen:
- EP-A- 0 923 389
- EP-A- 0 966 979
- EP-A- 1 000 958
- EP-A- 1 033 145
- EP-A- 1 419 793
- WO-A-2004/006885
- DE-B- 10 217 351
- DE-C- 10 217 350
- US-A- 4 950 258
- US-A- 5 741 323
- US-A- 6 160 084
- US-A1- 2002 142 119
- US-A1- 2003 153 971
- US-A1- 2004 098 108
- US-B1- 6 348 067
- US-B1- 6 388 043

## Beschreibung

Gegenstand der Erfindung ist ein temporärer Stent aus bioabbaubaren Shape Memory Polymeren (SMP) zum Einsatz im nicht-vaskulären oder vaskulären Bereich. Der Stent lässt sich in komprimierter Form minimalinvasiv implantieren und nimmt seine gewünschte Größe am Einsatzort durch den Shape-Memory-Effekt ein. Der Stent löst sich mit der Zeit durch biologischen Abbau langsam auf, wodurch ein weiterer Eingriff zur Entfernung entfällt.

### Stand der Technik

Zur Behandlung verstopfter oder verengter Hohlorgane oder nach operativen Eingriffen werden tubuläre Gewebestützen (Stents) in das Hohlorgan eingesetzt. Sie dienen dazu, die verengte Stelle aufzuhalten oder die Funktion des verletzten Hohlorgans zu übernehmen um wieder einen normalen Durch- oder Abfluss von Körperflüssigkeiten zu ermöglichen. Weiterhin werden Stents zur Behandlung verstopfter oder verengter Blutgefäße als Gefäßstützen eingesetzt, die die verengte Stelle aufhalten und wieder einen normalen Blutfluss ermöglichen.

Stents sind üblicherweise zylinderförmige Gebilde aus einer Art Maschendraht (wire coil design) oder Röhren, die perforiert oder nicht perforiert sein können (slottet tube design). Gebräuchliche Stents sind zwischen 1 und 12 cm lang und im Durchmesser etwa 1-12 mm groß.

Die mechanischen Anforderungen an einen Stent sind widersprüchlich. Einerseits muss ein Stent hohe radiale Kräfte auf das zu stützende Hohlorgan ausüben. Andererseits ist es erforderlich, dass sich der Stent radial komprimieren lässt, um ihn leicht in ein Hohlorgan einführen zu können, ohne dabei die Gefäßwand bzw. das umliegende Gewebe zu verletzen.

Dieses Problem wurde dadurch gelöst, dass die Stents in komprimierter Form eingesetzt und erst an der richtigen Stelle aufgespannt werden. Im komprimierten Zustand ist der Durchmesser kleiner als im expandierten Zustand. Dieser Vorgang lässt sich prinzipiell auch zur minimalinvasiven Entfernung des Stents nutzen. Ein mögliches Problem ist dabei jedoch, dass die üblicherweise eingesetzten metallischen Werkstoffe sich nicht immer vollständig gleichmäßig aufspannen bzw. wieder zusammenfalten lassen, was ein potentielles Verletzungsrisiko für das angrenzende Gewebe oder die Gefäßwand darstellt.

Zum minimalinvasiven Einsetzen eines Stents haben sich zwei verschiedene Technologien etabliert (Market report "US peripheral and vascular stent and AAA stent graft market" (Frost & Sullivan), 2001):
- Ballon expandierbare Stents (System besteht aus Ballon, Katheter, Stent)
- Selbst-expandierbare Stents (System besteht aus Hülse zum Einführen (protective sheeth), Katheter, Stent).

Selbst-expandierende Stents bestehen aus einem Formgedächtnismaterial (SM-Material), wobei metallische SM-Materialien, wie Nitinol im Vordergrund stehen. Der Formgedächtniseffekt ist ein in den letzten Jahren mit großem Interesse untersuchter Effekt, der eine gezielte Formveränderung durch Anlegen eines äußeren Reizes ermöglicht (zu Einzelheiten in dieser Hinsicht wird auf die bereits publizierte Literatur verwiesen, beispielsweise "Shape Memory Alloys", Scientific American, Vol. 281 (1979), Seiten 74 - 82). Die Materialien sind in der Lage bei einer Temperaturerhöhung ihre Form gezielt zu ändern. Der Formgedächtniseffekt wird ausgelöst, um den Durchmesser des Stents "von selbst" zu vergrößern und am Einsatzort zu fixieren.

Problematisch gestaltet sich, wie oben bereits angedeutet, die Entfernung expandierter Stents. Wenn der Stent aus einem röhrenförmigen Hohlraum herausgezogen werden muss, besteht die Gefahr, dass dabei das umliegende Gewebe durch Abrasion verletzt wird, weil der Stent zu groß ist und scharfe Kanten aufweist. Der Formgedächtniseffekt wird daher auch angewendet, um den Durchmesser des Stents wieder zu verringern, wenn ein Stent wieder entfernt werden soll. Beispiele für entfernbare Implantate (Stents) aus Formgedächtnis-Metallen sind im Stand der Technik bekannt: US 6413273 "Method and system for temporarily supporting a tubular organ"; US 6348067 "Method and system with shape memory heating apparatus for temporarily supporting a tubular organ"; US 5037427 "Method of implanting a stent within a tubular organ of a living body and of removing same"; US 5197978 "Removable heat-recoverable tissue supporting device".

Nitinol ist nicht einsetzbar bei einer Nickel-Allergie. Das Material ist außerdem sehr teuer und nur durch aufwendige Verfahren programmierbar. Dieses Programmierverfahren benötigt vergleichsweise hohe Temperaturen, so dass eine Programmierung im Körper nicht möglich ist. Das SM-Material wird daher außerhalb des Körpers programmiert, d.h. in seine temporäre Form gebracht. Nach dem Implantieren wird dann der Formgedächtniseffekt ausgelöst und der Stent expandiert, d.h. gewinnt seine permanente Form zurück. Eine Entfernung des Stents durch erneute Ausnutzung des Formgedächtniseffekts ist dann nicht möglich. Ein häufiges Problem bei metallischen Stents nicht nur im vaskularen Bereich ist darüber hinaus das Auftreten einer Restenose.

Andere metallische Stents aus SM-Materialien, beispielsweise aus der US 5197978, ermöglichen dagegen auch eine Ausnutzung des Formgedächtniseffekts zur Entfernung des Stents. Allerdings sind diese metallischen Werkstoffe in ihrer Herstellung sehr aufwendig und die Gewebeverträglichkeit ist nicht immer gesichert. Aufgrund der schlecht angepassten mechanischen Eigenschaften der Stents treten immer wieder Entzündungen und Schmerzen auf.

Der in US 5716410 "Temporary stent and method of use" beschriebene temporäre Stent ist eine Spirale aus einem Formgedächtniskunststoff (SMP). Das SMP-Material enthält einen eingebetteten Heizdraht. Der Heizdraht ist über einen Katheter-Schaft an einen elektrischen Kontroller angeschlossen, wobei das Schaftende als hohle Röhre über das eine Ende der Spirale gestülpt ist. Erwärmt man den implantierten Stent, der sich in seiner expandierten, temporären Form befindet, über die Schalttemperatur Tₜᵣₐₙₛ, so verkleinert sich der Durchmesser der Spirale. Hierdurch soll eine einfache Entfernung des Stents ermöglicht werden. Ein Nachteil der spiraligen Struktur besteht darin, dass die radialen Kräfte zu gering sind, um röhrenförmige Hohlräume aufzudehnen. Die radialen Kräfte der Spirale verteilen sich nur über eine geringe Kontaktflache zum Gewebe; es besteht die Gefahr einer lokalen mechanischen Überbelastung durch Druck, evtl. sogar einer Einschneidung ins Gewebe. Außerdem gestaltet sich die Befestigung des Katheter-Schafts (Heizelements) an den Heizdraht der implantierten Spirale als schwierig, weil der Katheter-Schaft erst über das eine Ende der Spirale übergestülpt werden muss.

In der US 4950258 wird eine Vorrichtung zur Aufdehnung eines verengten Blutgefäßes beschrieben. Diese besteht aus bioabbaubaren Polymeren basierend auf L-Lactid und / oder Glycolid und liegt in Form einer Spirale oder eines Röhrchens vor. Durch den Formgedächtniseffekt vergrößert sich der Durchmesser, so dass ein Gefäß aufgeweitet werden kann. Ein Nachteil der verwendeten Materialien ist deren Versprödung beim Abbau und die Entstehung von Partikeln, die losgelöst vom Device zu Gefäßverstopfungen führen können.

Die EP 1033145 beschreibt ebenfalls bioabbaubare Stents aus Formgedächtnispolymeren zum Einsatz in Blutgefäßen, Lymphgefäßen, im Gallengang oder im Harnleiter. Der Stent besteht aus einem Garn aus Homo- oder Copolymeren oder aus deren Blends basierend auf L-Lactid, Glycolid, E-Caprolacton, p-Dioxanon oder Trimethylencarbonat. Das Garn wird als Mono- oder Multifilament zu einer Maschenstruktur verwoben. Der Formgedächtniseffekt wird dazu genutzt, den Durchmesser des Stents zu vergrößern und am Einsatzort zu fixieren. Die Schalttemperatur ist eine Glastemperatur von nicht höher als 70 °C. Wirkstoffe oder Diagnostika können dem SMP zugemengt oder oberflächlich aufgetragen sein.

Die US 5964744 beschreibt Implantate wie z.B. Röhrchen und Katheter für den urogenitalen Bereich oder Magen-Darm Trakt aus polymeren Formgedächtnismaterialien, die ein hydrophiles Polymer enthalten. In einem wässrigen Medium nimmt das Material Feuchtigkeit auf, erweicht dadurch und ändert seine Form. Alternativ oder zusätzlich erweicht das Material beim Erwärmen. Beim uretheralen Stent wird der Effekt dazu genutzt, die geraden Enden des Stents am Einsatzort (z.B. Niere und Blase) zu verbiegen. Dadurch wird der uretherale Stent am Einsatzort fixiert, sodass der Stent bei peristaltischen Bewegungen des Gewebes nicht verrutschen kann.

Die WO 02/41929 beschreibt röhrenförmige Gefäßimplantate mit Formgedächtnis, die z.B. auch als Gallengangstents geeignet sind. Das Material ist ein aliphatisches, polycarbonatbasiertes thermoplastisches Polyurethan mit biostabiler Eigenschaft.

Ein Nachteil der im Stand der Technik verwendeten Materialien ist, dass sie nicht abbaubar sind. Das Implantat muss in einem zweiten Eingriff aus dem Körper entfernt werden.

Die US 6245103 beschreibt bioabsorbierbare, selbst-expandierende Stents aus geflochtenen Filamenten. Dabei wird ein Stent unter Anwendung einer äußeren radialen Kraft komprimiert. Der Stent ist auf einem Katheter montiert und wird von einer äußeren Hülse unter Spannung im komprimierten Zustand gehalten. Druckt man den Stent aus dieser Anordnung heraus, vergrößert sich sein Durchmesser von selbst aufgrund der Rückstellkraft des elastischen Materials. Hierbei handelt es sich nicht um den Formgedächtniseffekt, der durch einen externen Stimulus, z.B. eine Temperaturerhöhung, ausgelöst wird.

Die US 6569191 beschreibt selbst-expandierende Stents aus bioabbaubaren verwobenen Fäden. Außen auf dem Stent sind mehrere Streifen aus einem elastischen, bioabbaubaren Polymer festgeklebt. Die Streifen haben Formgedächtniseigenschaften. Sie ziehen sich beim Erwärmen auf Körpertemperatur oder durch Aufnahme von Feuchtigkeit zusammen. Dadurch wird der Stent ebenfalls zusammengezogen; gleichzeitig vergrößert sich der Durchmesser des Stents. Die elastischen Streifen verstärken die radialen Kräfte des Stents nach außen hin. Die Streifen bestehen beispielsweise aus einem Formgedächtnispolymer basierend auf Milchsäure und/oder Glycolsäure.

Die im Stand der Technik verwendeten bioabbaubaren, d.h. i.d.R. hydrolysierbaren Materialien zeigen jedoch teilweise ein problematisches Abbauverhalten. So findet ein Abbau statt, der zur Erzeugung von kleinen Partikeln führt, die ein potentielles Risiko darstellen. Die Partikel können die Kanäle oder Röhren (z.B. Harnröhre) verstopfen. Darüber hinaus kann ein Abbau auch die Struktur/Natur eines Implantats so ändern, dass eine Unverträglichkeit mit Blut und/oder Gewebe auftritt.

Weitere Probleme die immer wieder auftreten, sind Schmerzen, die durch die unzureichende mechanische Anpassung des Stents an das umliegende Gewebe verursacht werden, sowie das Verrutschen des Stents.

### Aufgabe der Erfindung

Da Stents aber einen immer weiteren Einsatzbereich in der Medizin erobert haben, sind Anstrengungen notwendig, die oben geschilderten Nachteile zu Überwinden. Es werden also Stents für den nicht-vaskularen oder vaskularen Einsatz gebraucht, die eine minimalinvasive Implantierung ebenso ermöglichen, wie einen Einsatz ohne dass ein Eingriff zur Entfernung notwendig ist, sowie einen biokompatiblen Abbau des Materials. Die Materialien für den Stent sollten darüber hinaus an den jeweiligen Einsatzort anpassbar sein, z.B. im Hinblick auf variierende mechanische Beanspruchungen. Die Materialien sollten bevorzugt noch eine weitere Funktionalisierung des Stents ermöglichen, z.B. durch Einbettung weiterer medizinisch nützlicher Stoffe.

Um die Nachteile des Standes der Technik zu überwinden wird benötigt:
- ein einfaches Verfahren welches die minimalinvasive Implantation eines Stents ermöglicht,
- ein Stent, der sich ohne Beeinträchtigung des umliegenden Gewebes abbaut, wobei gleichzeitig eine ausreichende mechanische Festigkeit über den beabsichtigten Einsatzzeitraum gesichert ist und wobei die Abbauprodukte keine nachteiligen Wirkungen ausüben,
- ein Verfahren zur Herstellung und Programmierung eines solchen Stents

### Kurze Beschreibung der Erfindung

Diese Aufgabe wird durch den Gegenstand der vorliegenden Erfindung gelöst, so wie er in den Ansprüchen definiert ist. Diese Stents umfassen ein bioabbaubares Formgedächtnis-Material (SMP-Material), wobei das SMP-Material unter kovalenten Polymernetzwerken ausgewählt ist und ein Multiblock-Copolymer umfassend Poly(pentadecalacton)-Blöcke enthält. Bevorzugt zeigt das SMP-Material einen thermisch induzierten oder Licht induzierten Formgedächtniseffekt. Die erfindungsgemäß, einzusetzenden SMP-Materialien können eine oder zwei Formen im Gedächtnis haben. Bevorzugte Ausführungsformen sind in den Unteransprüchen definiert.

Derartige Stents lösen die oben angesprochenen Probleme, entweder insgesamt oder doch zumindest teilweise. Somit stellt die vorliegende Erfindung Stents zur Verfügung, umfassend ein SMP-Material, die durch Einsatz des Formgedächtniseffekts minimalinvasiv und atraumatisch eingeführt werden können, die in ihrem Abbauverhalten gewebe- und ggf. hämokompatibel sind und eine ausreichende Festigkeit/Stabilität besitzen, so dass sie trotz des stattfindenden Abbaus eine ausreichende Stabilität zeigen. Derartige Stents, hergestellt mit den erfindungsgemäß, einzusetzenden Materialien zeigen insbesondere einen partikelfreies Abbauverhalten. Dies ist wichtig, da Partikel, die während eines Abbaus entstehen zu Problemen führen können, wie Verstopfung oder Verletzung von Harnleitern usw. Die Stents der vorliegenden Erfindung jedoch zeigen derartige Probleme nicht. Sollten beim Abbau trotzdem einmal Partikel entstehen, so waren diese relativ unproblematisch, da sie in der Form von Hydrogelpartikeln vorliegen, die weich und elastisch sind, so dass die oben geschilderten Probleme nicht auftreten.

Da die Stents in ihrer temporären Form vor der Platzierung im Körper vorliegen müssen sie bei ausreichend niedrigen Temperaturen bzw. ausreichend geschützt vor Bestrahlung gelagert werden, auch während des Transports, um ein ungewolltes Auslösen des Formgedächtniseffekts zu verhindern.

### Kurze Beschreibung der Figuren

Figur 1 zeigt schematisch den Größenunterschied zwischen der permanenten und der temporären Form des Stents der Erfindung.
Figur 2 zeigt eine schematische Darstellung der Arbeitsschritte zur Einbringung des Stents. Dabei stellt der hellgraue Teil den Stent, der dunkelgraue Teil den Ballon des Katheters und der schwarze Teil den Katheter dar.
Figur 3 zeigt schematisch ein bekanntes Verfahren zur Programmierung eines Stents (vgl. US 5591222).

### Detaillierte Beschreibung der Erfindung

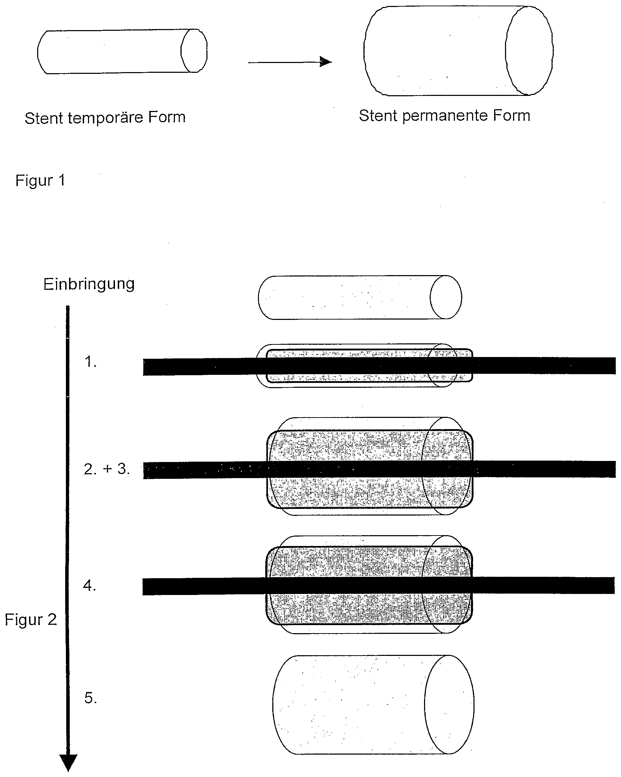

In bevorzugten Ausführungsformen wird die Aufgabe durch einen Stent aus SMP gelöst, wobei
- der Stent in seiner temporären Form auf einem temperierbaren oder mit einer geeigneten Lichtquelle ausgestattetem Ballonkatheter vormontiert ist,
- der Durchmesser der temporären Form kleiner ist als in der permanenten Form (vgl. Figur 1),
- die permanente Form als Gewebestütze fungiert,
- das SMP eine Schalttemperatur von 40 °C und höher oder einen Schaltwellenlängenbereich von 260 nm oder länger besitzt,
- der Stent in seiner komprimierten, temporären Form minimalinvasiv implantiert werden kann und seine gewünschte permanente Form erst gezielt durch den SM Effekt am Einsatzort einnimmt,
- die Erwärmung des Stents auf oder über seine Schalttemperatur entweder über eine Wärmequelle oder durch Bestrahlung mit IR- oder NIR-Licht oder durch Anlegen eines oszillierenden elektrischen Feldes erfolgen kann,
- für den Stent ein bioabbaubares SMP-Material verwendet wird, sodass eine nachträgliche Entfernung des Stents entfällt.

Ein mögliches Verfahren zum minimalinvasiven Einsetzen eines Stents, umfasst die folgenden Schritte (Figur 2)
1. der auf einem temperierbaren Ballonkatheter vorgesehene Stent wird in das tubuläre, nicht-vaskuläre Organ minimalinvasiv eingeführt
2. der platzierte Stent wird mittels Katheter über seine Tₜᵣₐₙₛ (mindestens 40 °C) erwärmt (Ballon füllt sich mit warmem Wasser (Flüssigkeit) oder Gas) oder mit Licht einer Wellenlange kleiner 260 nm bestrahlt. Hierbei expandiert der Stent und weitet sich
3. der Stent liegt nun in seiner permanenten Form vor (gedehnt) und der Ballonkatheter kann entfernt werden

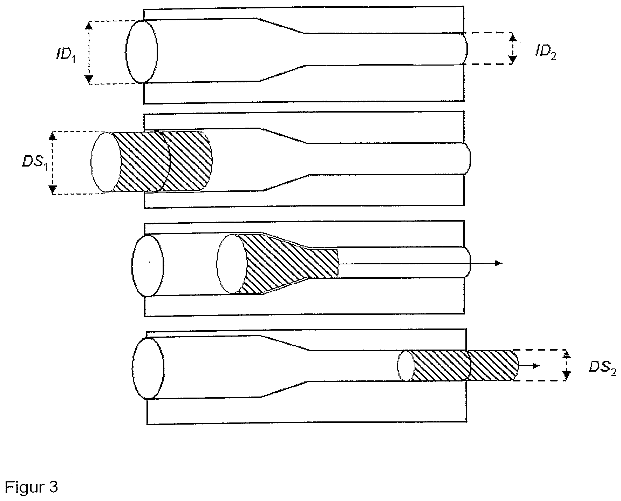

Verfahren zur Programmierung des erfindungsgemäßen Stents (Figur 3):
1. Der erfindungsgemäße Stent wird auf während der Programmierung auf einen Durchmesser gebracht der kleiner als der ursprüngliche Durchmesser ist. Hierzu wird eine geeignetes Werkzeug, das in Figur 3 dargestellt ist, verwendet. Dieses Programmierungswerkzeug besteht aus einem thermostatierbaren Block, welcher aus einer Röhre mit zwei unterschiedlichen Durchmessern (ID1 und ID2) enthält. Hierbei gelte ID₁ > ID₂.
2. Der Stent wird in seiner unprogrammierten (permanenten) Form in den linken Teil des Werkzeuges eingeführt. Hierbei soll der Außendurchmesser *DS*₁ des zu programmierenden Stents nur geringfügig kleiner sein als der Innendurchmesser *ID₁* des Werkzeuges.
3. Das Werkzeug nach Figur 3 wird auf eine Temperatur größer Tₜᵣₐₙₛ erwärmt.
4. Der auf eine Temperatur größer Tₜᵣₐₙₛ erwärmte Stent wird mit Hilfe eines Führungsdrahtes oder einer Führungsschnur in den rechten Bereich des Werkzeuges gezogen. Hierbei nimmt der Außendurchmesser des Stents auf *DS₂* ab und der Stent erhält seine temporäre Form.
5. Das Werkzeug nach Figur 3 wird auf eine Temperatur kleiner Tₜᵣₐₙₛ abgekühlt. Hierdurch wird die temporäre Form des Stents fixiert.
6. Der auf eine Temperatur kleiner Tₜᵣₐₙₛ gekühlte Stent wird mit Hilfe eines Führungsdrahtes oder einer Führungsschnur aus dem Werkzeug gezogen und kann auf einen geeigneten Katheter montiert werden.

Im Folgenden wird die vorliegende Erfindung weiter beschrieben.

Der Stent der vorliegenden Erfindung umfasst ein SMP-Material, das ein Polymernetzwerk ist und ein Multiblock-Copolymer umfassend Poly(pentadecalacton)-Blöcke enthält. Auch Komposite aus bioabbaubaren SMP mit anorganischen, abbaubaren Nanopartikeln sind geeignet. Bevorzugt ist in das SMP-Material kein Heizelement eingebettet. Der Formgedächtniseffekt kann thermisch mit Hilfe eines beheizbaren Mediums, durch Anwendung von IR- oder NIR-Strahlung, durch Anlegen eines oszillierenden elektrischen Feldes oder durch UV-Bestrahlung ausgelöst werden.

Mit der Definition, dass der erfindungsgemäße Stent ein SMP-Material umfasst soll definiert werden, dass der Stent einerseits im Wesentlichen aus einem SMP-Material besteht, aber dass andererseits der Stent auch ein Grundgerüst aus einem bioabbaubaren Kunststoff umfassen kann, eingebettet bzw. beschichtet mit einem SMP-Material. Diese beiden wesentlichen Konstruktionen bieten die folgenden Vorteile.

Stents, die im Wesentlichen aus SMP-Materialien bestehen verwenden das SMP-Material, um die mechanischen Eigenschaften des Stents zu bestimmen. Dadurch, dass die im Folgenden beschriebenen Materialien dazu eingesetzt werden, wird eine gute Gewebeverträglichkeit gesichert. Weiter können derartige Stents, wie oben beschrieben minimalinvasiv implantiert und wieder entfernt werden. Die SMP-Materialien können weiterhin relativ einfach verarbeitet werden, was die Herstellung erleichtert. Schließlich können die SMP-Materialien noch mit weiteren Stoffen compoundiert oder beschichtet werden, so dass eine weitere Funktionalisierung möglich ist. In diesem Zusammenhang wird auf die folgenden Ausführungen verwiesen.

Die zweite prinzipiell mögliche Ausführungsform ist ein Stent, der ein Grundgerüst umfasst, wie beispielsweise eine "Maschendrahtkonstruktion" oder eine verformbare Röhre. Diese Grundgerüste sind mit einem SMP-Material beschichtet bzw. in dieses eingebettet. Insbesondere mit Maschendrahtkonstruktionen hat es sich gezeigt, dass die SMP-Materialien eine ausreichend große Kraft ausüben können, um das Grundgerüst zu verformen, wenn der Formgedächtniseffekt ausgelöst wird. Diese Ausführungsform erlaubt es also die positiven Eigenschaften der konventionellen Stents mit den oben geschilderten positiven Effekten der SMP-Materialien zu kombinieren. Insbesondere können so Stents mit einer sehr hohen mechanischen Widerstandsfähigkeit erhalten werden, da das Grundgerüst dazu beiträgt. Daher eignet sich diese Ausführungsform insbesondere für Stents, die starker mechanischer Beanspruchung ausgesetzt werden. Weiterhin ermöglicht der Einsatz des Grundgerüsts die Einsatzmenge an SMP-Materialien zu verringern, was Kosten sparen kann.

Wenn das Grundgerüst aus einem metallischen Material besteht, dann bevorzugt aus bioabbaubaren Metallen wie Magnesium oder Magnesium-Legierungen.

Derartige Stents in Übereinstimmung mit der vorliegenden Erfindung ermöglichen eine sichere Platzierung des Stents und ein verträgliches Abbauverhalten. In einer Alternative zeigt der erfindungsgemäße Stent üblicherweise ein Verhalten, nach Platzierung, in Übereinstimmung mit einem 3-Phasen-Modell.

Die beabsichtigte Verwendung des Stents bestimmt dabei dessen Ausgestaltung, beispielsweise Oberflächenbeschaffenheit (Mikrostrukturierung) oder Vorliegen von Beschichtungen etc..

Prinzipiell sind dabei die folgenden Ausgestaltungen möglich.

Die Oberfläche des Stents ist kompatibel im Hinblick auf die physiologische Umgebung am Einsatzort ausgestaltet, durch geeignete Beschichtung (z.B. Hydrogel-Beschichtung) oder Oberflächenmikrostrukturierung. Beim Stent-Design müssen die Rahmenbedingungen wie pH Wert und Keimzahl je nach Einsatzort berücksichtigt werden. Anschließend erfolgt eine Besiedelung der Oberfläche mit Endothelzellen, was ggf. durch eine entsprechende Modifikation der Oberfläche (z.B. Beschichtung) unterstutzt werden kann. Damit wird der Stent langsam mit Endothelzellen überwachsen.

Bei vaskulären Stents ist die Oberfläche des Stents hämokompatibel ausgestaltet, durch geeignete Beschichtung (z.B. Hydrogel-Beschichtung) oder Oberflächenmikrostrukturierung, so dass der Stent die vergleichsweise kurze Zeit nach Platzierung im vollen Blutkontakt ohne Beeinträchtigung des Organismus ermöglicht. Anschließend erfolgt, wie oben ausgeführt, die Besiedelung der Oberfläche, so dass der Stent langsam in die Gefäßwand aufgenommen wird.

Schließlich setzt der üblicherweise hydrolytische Abbau ein, der Stent degradiert im Kontakt mit dem Weichgewebe aber übt aufgrund des oben beschriebenen Abbauverhaltens (partikelfreier Abbau, mechanische Stabilität wird durch Abbau über eine lange Zeit nicht beeinträchtigt) weiter die gewünschte Stützwirkung aus.

Eine andere Alternative ist, dass der Stent nach Platzierung außerhalb der Endothelschicht verbleiben soll, was durch geeignete Maßnahmen, wie Auswahl der Oberfläche, Segmentauswahl für die SMP-Materialien etc. erreicht werden kann.

Im Folgenden werden geeignete Materialien für die Stents der vorliegenden Erfindung beschrieben.

SMP-Materialien im Sinne der vorliegenden Erfindung sind Materialien, die durch ihre chemisch-physikalische Struktur in der Lage sind, gezielte Formänderungen durchzuführen. Die Materialien besitzen neben ihrer eigentlichen permanenten Form eine weitere Form, die dem Material temporär aufgeprägt werden kann. Solche Materialien sind durch zwei strukturelle Merkmale charakterisiert: Netzpunkte (physikalisch oder kovalent) und Schaltsegmente.

SMP mit thermisch induziertem Formgedächtniseffekt besitzen mindestens ein Schaltsegment mit einer Übergangstemperatur als Schalttemperatur. Die Schaltsegmente bilden temporäre Vernetzungsstellen, die sich beim Erwärmen oberhalb der Übergangstemperatur lösen und beim Abkühlen erneut bilden. Die Übergangstemperatur kann eine Glastemperatur T_{g} amorpher Bereiche oder Schmelztemperatur Tₘ kristalliner Bereiche sein. Sie wird im Folgenden verallgemeinert als Tₜᵣₐₙₛ bezeichnet.

Oberhalb van Tₜᵣₐₙₛ befindet sich das Material im amorphen Zustand und ist elastisch. Wird also eine Probe über die Übergangstemperatur Tₜᵣₐₙₛ erwärmt, im flexiblen Zustand dann deformiert und wieder unter die Übergangstemperatur abgekühlt, so werden die Kettensegmente durch Einfrieren von Freiheitsgraden im deformierten Zustand fixiert (Programmierung). Es werden temporäre Vernetzungsstellen (nichtkovalent) geformt, so dass die Probe auch ohne äußere Last nicht mehr in ihre ursprüngliche Form zurück kehren kann. Beim erneuten Erwärmen auf eine Temperatur oberhalb der Übergangstemperatur werden diese temporären Vernetzungsstellen wieder aufgelöst und die Probe kehrt zu ihrer ursprünglichen Form zurück. Durch erneutes Programmieren kann die temporäre Form wieder hergestellt werden. Die Genauigkeit, mit der die ursprüngliche Form wieder erhalten wird, wird als Rückstellverhältnis bezeichnet.

In photoschaltbaren SMP übernehmen photoreaktive Gruppen, die sich durch Bestrahlung mit Licht reversibel miteinander verknüpfen lassen, die Funktion des Schaltsegments. Die Programmierung einer temporären Form und Wiederherstellung der permanenten Form erfolgt in diesem Fall durch Bestrahlung, ohne dass eine Temperaturänderung erforderlich ist.

Zur Herstellung der erfindungsgemäßen Stents können thermoplastische Elastomere verwendet werden. Geeignete thermoplastische Elastomere zeichnen sich durch mindestens zwei Übergangstemperaturen aus. Die höhere Übergangstemperatur kann den physikalischen Netzpunkten, die die permanente Form des Stents bestimmen, zugeordnet werden. Die niedrigere Übergangstemperatur, bei welcher der Formgedächtniseffekt ausgelöst werden kann, kann den Schaltsegmenten zugeordnet werden (Schalttemperatur, Tₜᵣₐₙₛ). Bei geeigneten thermoplastischen Elastomeren liegt die Schalttemperatur typischerweise etwa 3 bis 20 °C oberhalb der Körpertemperatur.

Beispiele für thermoplastische Elastomere sind Multiblockcopolymere. Erfindungsgemäß Multiblockcopolymere sind aus den Blöcken (Makrodiolen) zusammengesetzt, die erfindungsgemäß Poly(pentadecalacton)-Blöcke enthalten. Weitere bevorzugte Blöcke sind ausgewählt aus α,ω-Diol-Polymeren von Poly(ε-caprolacton) (PCL), Poly(ethylen glycol) (PEG), Poly(ethylenoxid), Poly(propylenoxid), Poly(propylenglycol), Poly(tetrahydrofuran), Poly(dioxanon), Poly(lactid), Poly(glycolid) und Poly(lactid-ran-glycolid) oder aus α,ω-Diol-Copolymeren der Monomere, auf denen die oben genannten Verbindungen basieren, in einem Molekulargewichtsbereich Mₙ von 250 bis 500 000 g/mol. Zwei unterschiedliche Makrodiole werden mit Hilfe eines geeigneten bifunktionellen Kopplungsreagenz (Im Speziellen ein aliphatisches oder aromatisches Diisocyanat oder Disäurechlorid oder Phosgen) zu einem thermoplastischen Elastomer mit Molekulargewichten Mₙ im Bereich von 500 bis 50 000 000 g/mol verknüpft. In einem phasensegregierten Polymer kann bei jedem der Blöcke des o. g. Polymers unabhängig vom anderen Block eine Phase mit mindestens einem thermischen Übergang (Glas- oder Schmelzübergang) zugeordnet werden.

Besonders bevorzugt sind Multiblockcopolymere aus Makrodiolen basierend auf Pentadecalacton (POL) und ε-Caprolacton (PCL) und einem Diisocyanat. Die Schalttemperatur - hier eine Schmelztemperatur - kann über die Blocklänge des PCLs im Bereich zwischen ca. 30 und 55 °C eingestellt werden. Die physikalischen Netzpunkte zur Fixierung der permanenten Form des Stents werden von einer zweiten kristallinen Phase mit einem Schmelzpunkt im Bereich von 87 - 95 °C gebildet. Auch Blends aus Multiblockcopolymeren sind geeignet. Durch das Mischungsverhältnis lassen sich die Übergangstemperaturen gezielt einstellen.

Zur Herstellung der erfindungsgemäßen Stents können auch Polymernetzwerke verwendet werden. Geeignete Polymernetzwerke zeichnen sich durch kovalente Netzpunkte und mindestens einem Schaltsegment mit mindestens einer Übergangstemperatur aus. Die kovalenten Netzpunkte bestimmen die permanente Form des Stents. Bei geeigneten Polymernetzwerken liegt die Schalttemperatur, bei welcher der Formgedächtniseffekt ausgelöst werden kann, typischerweise etwa 3 bis 20 °C oberhalb der Körpertemperatur.

Zur Herstellung eines kovalenten Polymernetzwerks wird eines der im obigen Abschnitt beschriebenen Makrodiole mit Hilfe eines multifunktionellen Kopplungsreagenz vernetzt. Dieses Kopplungsreagenz kann eine mindestens trifunktionelle, niedermolekulare Verbindung oder ein multifunktionales Polymer sein. Im Falle des Polymers kann es sich um ein Sternpolymer mit mindestens drei Armen, ein graft-Polymer mit mindestens zwei Seitenketten, ein hyperverzweigtes Polymer oder um eine dendritische Struktur handeln. Sowohl im Falle der niedermolekularen als auch der polymeren Verbindungen müssen die Endgruppen zur Reaktion mit den Diolen befähigt sein. Im Speziellen können hierfür Isocyanatgruppen verwendet werden (Polyurethan-Netzwerke).

Insbesondere bevorzugt sind amorphe Polyurethannetzwerke aus Triolen und/oder Tetrolen und Diisocyanat. Die Darstellung sternförmiger Präpolymere erfolgt durch die Copolymerisation von Monomeren mit hydroxyfunktionellen Initiatoren unter Zusatz des Katalysators Dibutylzinn(IV)oxid (DBTO). Als Initiatoren der ringöffnenden Polymerisation werden Ethylenglykol, 1,1,1-Tris(hydroxy-methyl)ethan bzw. Pentaerythrit eingesetzt. Die erfindungsgemäßen Netzwerke können einfach durch Umsetzung der Präpolymere mit Diisocyanat, z.B. einem Isomerengemisch aus 2,2,4- und 2,4,4-Trimethylhexan-1,6-diisocyanat (TMDI), in Lösung, z.B. in Dichloromethan, und anschließender Trocknung erhalten werden.

Weiterhin können die im obigen Abschnitt beschriebenen Makrodiole zu entsprechenden α,ω-Divinylverbindungen funktionalisiert werden, die thermisch oder photochemisch vernetzt werden können. Die Funktionalisierung erlaubt bevorzugt eine kovalente Verknüpfung der Makromonomere durch Reaktionen, die keine Nebenprodukte ergeben.

Bevorzugt wird diese Funktionalisierung durch ethylenisch ungesättigte Einheiten zur Verfügung gestellt, insbesondere bevorzugt durch Acrylatgruppen und Methacrylatgruppen, wobei letztere insbesondere bevorzugt sind. Hierbei kann Im Speziellen die Umsetzung zu α,ω-Makrodimethacrylaten, bzw. Makrodiacrylaten durch die Reaktion mit den entsprechenden Säurechloriden in Gegenwart einer geeigneten Base durchgeführt werden. Die Netzwerke werden erhalten durch das Vernetzen der endgruppenfunktionalisierten Makromonomere. Diese Vernetzung kann erreicht werden durch das Bestrahlen der Schmelze, umfassend die endgruppenfunktionalisierte Makromonomerkomponente und ggf. ein niedermolekulares Comonomer, wie nachfolgend erläutert wird. Geeignete Verfahrensbedingungen dafür sind das Bestrahlen der Mischung in Schmelze, vorzugsweise bei Temperaturen im Bereich van 40 bis100 °C, mit Licht einer Wellenlange von vorzugsweise 308 nm. Alternativ ist eine Wärmevernetzung möglich wenn ein entsprechendes Initiatorsystem eingesetzt wird.

Werden die oben beschriebenen Makromonomere vernetzt, so entstehen Netzwerke mit einer einheitlichen Struktur, wenn lediglich eine Art an Makromonomer eingesetzt wird. Werden zwei Arten an Monomeren eingesetzt, so werden Netzwerke vom AB-Typ erhalten. Solche Netzwerke vom AB-Typ können auch erhalten werden, wenn die funktionalisierten Makromonomere mit geeigneten niedermolekularen oder oligomeren Verbindungen copolymerisiert werden. Sind die Makromonomere mit Acrylatgruppen oder Methacrylatgruppen funktionalisiert, so sind geeignete Verbindungen, die copolymerisiert werden können, niedermolekulare Acrylate, Methacrylate, Diacrylate oder Dimethacrylate. Bevorzugte Verbindungen dieser Art sind Acrylate, wie Butylacrylat oder Hexylacrylat, und Methacrylate, wie Methylmethacrylat und Hydroxyethylmethacrylat.

Diese Verbindungen, die mit den Makromonomeren copolymerisiert werden können, können in einer Menge von 5 bis 70 Gew.-%, bezogen auf das Netzwerk aus Makromonomer und der niedermolekularen Verbindung vorliegen, bevorzugt in einer Menge von 15 bis 60 Gew.-%. Der Einbau von variierenden Mengen der niedermolekularen Verbindung erfolgt durch Zugabe entsprechender Mengen an Verbindung zur zu vernetzenden Mischung. Der Einbau der niedermolekularen Verbindung in das Netzwerk erfolgt in einer Menge, die der in der Vernetzungsmischung enthaltenen Menge entspricht.

Die erfindungsgemäß zu verwendenden Makromonomere werden im Folgenden detailliert beschrieben.

Durch Variation des Molgewichtes der Makrodiole lassen sich Netzwerke mit unterschiedlichen Vernetzungsdichten (bzw. Segmentlangen) und mechanischen Eigenschaften erzielen. Die kovalent zu vernetzenden Makromonomere weisen bevorzugt ein Zahlenmittel des Molgewichts, bestimmt durch GPC-Analyse von 2000 bis 30000 g/mol, bevorzugt von 5000 bis 20000 g/mol und insbesondere bevorzugt von 7500 bis 15000 g/mol auf. Die kovalent zu vernetzenden Makromonomere weisen bevorzugt an beiden Enden der Makromonomerkette eine Methacrylatgruppe auf. Eine derartige Funktionalisierung erlaubt die Vernetzung der Makromonomere durch einfache Photoinitiation (Bestrahlung).

Neben den Poly(pentadecalacton)-Makromonomeren sind weitere Polyestermakromonomere bevorzugt, insbesondere Polyestermakromonomere auf der Basis von ε-Caprolacton. Andere mögliche Polyestermakromonomere basieren auf Lactideinheiten, Glycolideinheiten, p-Dioxanoneinheiten und deren Mischungen und Mischungen mit ε-Caprolactoneinheiten, wobei Polyestermakromonomere mit Caprolactoneinheiten insbesondere bevorzugt sind. Bevorzugte Polyestermakromonomere sind weiterhin Poly(caprolacton-*co*-glycolid) und Poly(caprolacton-*co*-lactid). Über das Mengenverhältnis der Comonomere lässt sich die Übergangstemperatur einstellen, ebenso wie Abbaugeschwindigkeit.

Insbesondere bevorzugt sind die erfindungsgemäß einzusetzenden Makromonomere Polyester, umfassend die vernetzbaren Endgruppen. Ein insbesondere bevorzugter, erfindungsgemäß einzusetzender Polyester ist ein Polyester auf der Basis von ε-Caprolacton oder Pentadecalacton, für den die oben aufgeführten Angaben über das Molgewicht gelten. Die Herstellung eines solchen Polyestermakromonomers, an den Enden funktionalisiert, bevorzugt mit Methacrylatgruppen, kann durch einfache Synthesen, die dem Fachmann bekannt sind, hergestellt werden. Diese Netzwerke, ohne Berücksichtigung der weiteren wesentlichen polymeren Komponente der vorliegenden Erfindung, zeigen semikristalline Eigenschaften und weisen einen Schmelzpunkt der Polyesterkomponente auf (bestimmbar durch DSC-Messungen), der abhängig von der Art der eingesetzten Polyesterkomponente ist und darüber somit auch steuerbar ist. Bekanntermaßen liegt diese Temperatur (Tₘ¹) für Segmente basierend auf Caprolactoneinheiten zwischen 30 und 60 °C in Abhängigkeit von der Molmasse des Makromonomers.

Zur Herstellung von Stents mit zwei Formen im Gedächtnis sind Netzwerke mit zwei Übergangstemperaturen geeignet, wie beispielsweise interpenetrierende Netzwerke (IPNs). Das kovalente Netzwerk basiert auf Poly(caprolacton)-dimethacrylat als Makromonomer; die interpenetrierende Komponente ist ein Multiblockcopolymer aus Makrodiolen basierend auf Pentadecalacton (PDL) und ε-Caprolacton (PCL) und einem Diisocyanat. Die permanente Form des Materials wird durch die kovalenten Netzpunkte bestimmt. Die beiden Übergangstemperaturen - Schmelztemperaturen der kristallinen Phasen - lassen sich als Schalttemperaturen für jeweils eine temporäre Form nutzen. Die untere Schalttemperatur Tₜᵣₐₙₛ 1 kann über die Blocklänge des PCLs im Bereich zwischen ca. 30 und 55 °C eingestellt werden. Die obere Schalttemperatur Tₜᵣₐₙₛ 2 liegt im Bereich von 87 - 95 °C.

Weiterhin können zur Herstellung der erfindungsgemäßen Stents photosensitive Netzwerke verwendet werden. Geeignete photosensitive Netzwerke sind amorph und zeichnen sich durch kovalente Netzpunkte aus, die die permanente Form des Stents bestimmen. Ein weiteres Merkmal ist eine photoreaktive Komponente bzw. eine durch Licht reversibel schaltbare Einheit, die die temporäre Form des Stents bestimmt.

Im Falle der photosensitiven Polymere wird ein geeignetes Netzwerk verwendet, welches entlang der amorphen Kettensegmente photosensitive Substituenten enthält. Bei UV-Bestrahlung sind diese Gruppen fähig, kovalente Bindungen miteinander einzugehen. Deformiert man das Material und bestrahlt es mit Licht einer geeigneten Wellenlange λ1, wird das ursprüngliche Netzwerk zusätzlich quervernetzt. Aufgrund der Vernetzung erreicht man eine temporäre Fixierung des Materials im deformierten Zustand (Programmierung). Da die Photovernetzung reversibel ist, lässt sich durch erneutes Bestrahlen mit Licht einer anderen Wellenlange λ2 die Vernetzung wieder lösen und somit die ursprüngliche Form des Materials wieder abrufen (Wiederherstellung). Ein solcher photomechanischer Zyklus lässt sich beliebig oft wiederholen. Die Basis der photosensitiven Materialien ist ein weitmaschiges Polymernetzwerk, das, wie vorstehend ausgeführt, transparent im Hinblick auf die zur Auslösung der Formveränderung gedachten Strahlung ist, d.h. bevorzugt eine UV-transparente Matrix bildet.

Als Comonomer zur Herstellung der polymeren Netzwerke der vorliegenden Erfindung wird eine Komponente eingesetzt, die für die Vernetzung der Segmente verantwortlich ist. Die chemische Natur dieser Komponente hängt natürlich von der Natur der Monomere ab.

Als weitere Komponente umfasst das erfindungsgemäße Netzwerk eine photoreaktive Komponente (Gruppe), die für die Auslosung der gezielt steuerbaren Formveränderung mitverantwortlich ist. Diese photoreaktive Gruppe ist eine Einheit, die durch Anregung mit einer geeigneten Lichtstrahlung, bevorzugt UV-Strahlung zu einer reversiblen Reaktion fähig ist (mit einer zweiten photoreaktiven Gruppe), die zur Erzeugung oder Lösung von kovalenten Bindungen führt. Bevorzugte photoreaktive Gruppen sind solche, die zu einer reversiblen Photodimerisierung fähig sind. Als photoreaktive Komponenten in den erfindungsgemäßen photosensitiven Netzwerken dienen bevorzugt verschiedene Zimtsäureester (Cinnamate, CA) und Cinnamylacylsäureester (Cinnamylacylate, CAA).

Es ist bekannt, dass Zimtsäure und ihre Derivate unter UV-Licht von etwa 300 nm unter Ausbildung eines Cyclobutans dimerisieren. Die Dimere lassen sich wieder spalten, wenn mit UV-Licht einer kleineren Wellenlange von etwa 240 nm bestrahlt wird. Die Absorptionsmaxima lassen sich durch Substituenten am Phenylring verschieben, verbleiben aber stets im UV-Bereich. Weitere Derivate, die sich photodimerisieren lassen, sind 1,3-Diphenyl-2-propen-1-on (Chalkon), Cinnamylacylsäure, 4-Methylcoumarin, verschiedene ortho-substituierte Zimtsäuren, Cinammyloxysilane (Silylether des Zimtalkohols).

Bei der Photodimerisierung von Zimtsäure und ähnlichen Derivaten handelt es sich um eine [2+2] Cycloaddition der Doppelbindungen zu einem Cyclobutanderivat. Sowohl die E- als auch Z-Isomere sind in der Lage, diese Reaktion einzugehen. Unter Bestrahlung läuft die E/Z-Isomerisierung in Konkurrenz zur Cycloaddition ab. Im kristallinen Zustand ist die E/Z-Isomerisierung jedoch gehindert. Aufgrund der verschiedenen Anordnungsmöglichkeiten der Isomere zueinander sind theoretisch 11 verschiedene stereoisomere Produkte (Truxillsäuren, Truxinsäuren) möglich. Der für die Reaktion erforderliche Abstand der Doppelbindungen zweier Zimtsäuregruppen beträgt etwa 4 Å.

Die Netzwerke zeichnen sich durch die folgenden Eigenschaften aus:
Insgesamt sind die Netzwerke gute SMP-Materialien, mit hohen Rückstellwerten, d.h. die ursprüngliche Form wird auch bei mehrfachem Durchlaufen eines Zyklus an Formänderungen zu einem hohen Prozentsatz, üblicherweise oberhalb von 90%, erneut erhalten. Dabei tritt auch kein nachteiliger Verlust an mechanischen Eigenschaftswerten auf.

Da die oben genannten Materialien auf aliphatischen Polyestern basieren, sind die eingesetzten SMP-Materialien hydrolysierbar bzw. bioabbaubar. Überraschender Weise hat sich gezeigt, dass diese Materialien sich einerseits in biokompatibler Weise zersetzen (d.h. die Abbauprodukte sind nicht toxisch) und dabei gleichzeitig während des Abbauvorgangs die mechanische Integrität des Stents erhalten bleibt, was eine ausreichend lange Funktionalität des Stents sichert.

Zur Steigerung der Hämokompatibilität kann die chemische Struktur der erfindungsgemäß eingesetzten SMP-Materialien modifiziert werden, z.B. durch den Einbau der oben genannten Poly- oder Oligoethereinheiten.

### Verarbeitung der Polymere zu Stents

Zur Verarbeitung von thermoplastischen Elastomeren zu Stents beispielsweise in Form einer Hohlröhre o. ä. (Figur 1) können alle üblichen polymertechnischen Methoden wie Spritzguss, Extrusion, Rapid Prototyping u.s.w. angewandt werden, die dem Fachmann bekannt sind. Zusätzlich können Fertigungsverfahren wir Laser-Cutting eingesetzt werden. Im Falle der thermoplastischen Elastomere können verschiedene Designs durch ein Ausspinnen in Mono- oder Multifilament-Faden mit anschließender Verwebung zu einem zylinderförmigen Netz mit Maschenstruktur realisiert werden.

Bei der Herstellung von Stents aus Polymernetzwerken muss beachtet werden, dass die Form in der die Vernetzungsreaktion der Makromonomere erfolgt, der permanenten Form des Stents entspricht (Formgussverfahren mit anschließender Härtung). Speziell die erfindungsgemäßen Netzwerkmaterialen bedürfen daher zur weiteren Verarbeitung spezieller Fräs- und Schneidemethoden. Hierbei empfiehlt sich die Perforation bzw. das Schneiden beispielsweise einer Röhre mit Hilfe von LASER-Licht geeigneter Wellenlänge. Mit Hilfe dieser Technik - Im Speziellen bei der Kombination von CAD und gepulsten CO₂ oder YAG-Lasern - können Formen bis zu einer Größe von 20 µm herab gearbeitet werden, ohne dass das Material einer hohen thermischen Belastung (und damit unerwünschten Nebenreaktionen an der Oberfläche) ausgesetzt wird. Alternativ empfiehlt sich eine spanabhebende Weiterverarbeitung zum einsatzfähigen Stent.

Die zweite Ausführungsform wird erhalten durch Beschichten bzw. Einbetten eines konventionellen Materials (siehe oben) in ein SMP-Material durch ein geeignetes Verfahren.

Die erforderlichen mechanischen Eigenschaften eines Stents hängen vom Einsatzort ab und erfordern ein angepasstes Design. Wird der implantierte Stent starken mechanischen Verformungen ausgesetzt, ist eine sehr hohe Flexibilität erforderlich, ohne dass der Stent bei den Bewegungen kollabiert. Prinzipiell ist hier das "wire coil design" besser geeignet. In anderen Bereichen tiefergelegener Organe, wird der Stent weniger durch Verformungen mechanisch belastet, sondern eher durch einen relativ hohen Außendruck. Ein hierfür geeigneter Stent muss sich durch hohe radiale Kräfte auf das umliegende Gewebe auszeichnen. Hier erscheint das "slotted tube design" besser geeignet. Röhren mit Perforationen ermöglichen den Einstrom von Flüssigkeiten aus dem umliegenden Gewebe in den Stent (Drainage).

Da bei Stents die im nicht-vaskularen Bereich eingesetzt werden sollen, Drainage-Effekte im Vordergrund stehen, bietet sich für derartige Stents insbesondere ein Design mit eingebettetem konventionellen Grundgerüst an, oder ein Design im Wesentlichen aus SMP-Material (perforierte Röhre oder Netzkörper), da bei diesen am einfachsten die für die Drainage notwendige Durchlässigkeit für Flüssigkeit gegeben ist, bei gleichzeitig ausreichender mechanischer Festigkeit.

Insbesondere im Stand der Technik gab es immer wieder Probleme bei Blutgefäßen mit kleinen Durchmessern, da die bekannten Stents für solche Gefäße zu wenig flexibel und anpassbar sind. Die Stents der vorliegenden Erfindung jedoch ermöglichen auch einen sicheren Einsatz in solchen Gefäßen, da die überlegenen elastischen Eigenschaften der SMP-Materialien, d.h. hohe Elastizität bei kleinen Auslenkungen und hohe Festigkeit bei großer Ausdehnung, das Gefäß schützt, beispielsweise bei pulsatilen Bewegungen der Arterien.

### Funktionalisierung der Stents

Zur leichteren Einführung des Stents kann dieser ggf. mit einem Coating, das die Gleitfähigkeit erhöht, ausgerüstet werden (z.B. Silicone oder Hydrogele).

Weitere Möglichkeiten zur Verbesserung der Hämokompatibilität umfassen die Möglichkeit, dass eine Beschichtung vorgesehen wird (die dazu erforderlichen Materialien sind dem Fachmann bekannt) oder es kann eine Mikrostrukturierung der Oberfläche vorgenommen werden. Geeignete Verfahren zur Oberflächenmodifikation sind beispielsweise die Plasmapolymerisation und Pfropfpolymerisation.

Zur leichteren Lokalisierung des Stents durch bildgebende diagnostische Verfahren kann der Formgedächtniskunststoff mit einem geeigneten Rontgen-Kontrastmittel (bspw. BaSO₄) verblendet werden. Eine weitere Möglichkeit besteht im Einbau von Metallfäden (bspw. Edelstahl) in den Stent. Diese Metallfäden dienen hierbei nicht zur Stabilisierung (sondern zur Lokalisierung); es ist deren ausschließliche Aufgabe den Röntgenkontrast zu erhöhen. Eine dritte Möglichkeit besteht in der Verblendung mit Metallen, die neben ihres hohen Röntgenkontrasts noch virostatische, fungizide oder bakterizide Eigenschaften (bspw. Nano-Silber) besitzen. Eine weitere Alternative in dieser Hinsicht ist der Einbau von röntgenopaken Chromophoren wie z.B. Trijodbenzol-Derivate in die SMP-Materialien selbst.

In einer weiteren Ausführungsform kann das SMP mit anorganischen, bioabbaubaren Nanopartikeln compoundiert werden. Beispiele sind Partikel aus Magnesium oder Magnesium-Legierungen oder Magnetit. Geeignet sind auch Partikel aus Carbon. Derartig funktionalisierte SMP können in einem oszillierenden elektrischen Feld aufgeheizt werden, um den Formgedächtniseffekt auszulösen.

Der erfindungsgemäße Stent kann weiterhin mit einer Reihe von therapeutisch wirksamen Substanzen beladen sein, welche den Heilungsprozess unterstützen, die Restenose des Stents unterdrücken oder auch Folgeerkrankungen verhindern. Im Speziellen können eingesetzt werden:
- Entzündungshemmende Wirkstoffe (bspw. Ethacridinlactat)
- Schmerzlindernde Wirkstoffe (bspw. Acetylsalicilsäure)
- Antibiotische Wirkstoffe (bspw. Enoxacin, Nitrofurantoin)
- Wirkstoffe gegen Viren, Pilze (bspw. Elementares Silber)
- Antithrombische Wirkstoffe (bspw. AAS, Clopidogrel, Hirudin, Lepirudin, Desirudin)
- Cytostatische Wirkstoffe (bspw. Sirolimus, Rapamycin, oder Rapamune)
- Immunosuppressive Wirkstoffe (bspw. ABT-578)
- Wirkstoffe zur Herabsetzung der Restenose (bspw. Taxol, Paclitaxel, Sirolimus, Actinomycin D)

Der erfindungsgemäße Stent kann auf unterschiedliche Art und Weise mit Wirkstoffen beladen werden.

Die Wirkstoffe können entweder direkt mit dem Kunststoff verblendet oder als Coating auf den Stent aufgebracht werden.

Derartige Stents können auch im Bereich Gentherapie eingesetzt werden.

Werden die Wirkstoffe in die hydrophile Beschichtung eingebracht, so. werden diese freigesetzt, so lange der Stent eine diffusionskontrollierte Freisetzung ermöglicht. Hierbei ist zu beachten, dass die Diffusionsgeschwindigkeit der Wirkstoffe aus der hydrophilen Beschichtung höher sein muss als die Abbaugeschwindigkeit des Materials des Stents.

Werden die Wirkstoffe in das Material des erfindungsgemäßen Stents eingebracht, so erfolgt die Freisetzung der Wirkstoffe während des Abbaus, ggf. nachdem der Stent mit Endothelzellen überwachsen ist und im Kontakt mit dem Weichgewebe steht. Hierbei geht die Freisetzung des Wirkstoffes mit dem Abbau des Stents einher; daher ist zu beachten, dass die Diffusionsgeschwindigkeit des Wirkstoffes aus dem Stent geringer sein muss als die Abbaugeschwindigkeit des Materials des Stents.

Für vaskulare Stents gilt dabei folgendes:
Werden die Wirkstoffe in die hydrophile Beschichtung eingebracht, so werden diese freigesetzt, so lange der Stent in Kontakt fließendem Blut steht. Hierbei ist zu beachten, dass die Diffusionsgeschwindigkeit der Wirkstoffe aus der hydrophilen Beschichtung höher sein muss als die Abbaugeschwindigkeit des Materials des Stents.

Im Speziellen kommen folgende Einsatzgebiete in Frage

### Iliac stents

Diese sind 10-120 mm lang, meist 40-60 mm. Werden im Bauchbereich eingesetzt. Oft werden üblicherweise 2 Stents verwendet, da der Einsatz von langen Stents schwierig ist. Die Stents der vorliegenden Erfindung zeichnen sich jedoch durch eine gute Flexibilität aus und ermöglichen eine sehr schonende minimalinvasive Applikation und Entfernung, so dass die Stents der vorliegenden Erfindung auch in Längen eingesetzt werden können, die im Stand der Technik für nicht machbar gehalten werden.

### Renal stents

Hier ist eine hohe radiale Stärke erforderlich, wegen hoher elastischer Belastung in der Nieren-Arterie, die gegebenenfalls eine erhöhte mechanische Verstärkung des Stents nötig macht. Hier ist das "slotted tube Design" geeignet. Diese Ausführungsform erlaubt die Verwendung von radioopaquen Markern. Hier kommt es weiter darauf an, eine sichere Installation des Stents auf dem Ballon des Katheters und eine Präzision beim Einsetzen zu gewährleisten. Aufgrund unterschiedlicher Anatomie der Lebewesen sind hier angepasste, variable Längen und Durchmesser notwendig. Weiterhin empfiehlt sich die Kombination mit distalem Schutzdevice oder einem Plaque-Filter.

### Karotis-Stents (Halsschlagader)

- Ein langer Stent kann hier eingesetzt werden, um die bisherige Technik der Kombination zweier Stents zu vermeiden.
- ist auch an Gefäßgabelungen einsetzbar
- Optimale Anpassung an verschiedene Durchmesser möglich
- Netzwerk mit engen Maschen wünschenswert und realisierbar (s.o.), da Filterfunktion u. U. notwendig zur Vermeidung des Eintrags von Blutgerinnsel ins Gehirn (Plaque-Filterfunktion).
- Stent muss druckstabil sein, das u. U. von außen Druck aufgebaut werden könnte, dabei soll der Stent nicht kollabieren;

### Femoral-poplietal Stents (Hüfte-Knie)

Hier ist eine hohe radiale Stärke wegen hoher elastischer Belastung im Blutgefäß, die gegebenenfalls eine erhöhte mechanische Verstärkung des Stents nötig macht. Hier ist das "slotted tube Design" eher geeignet insbesondere kann hier auch die Verwendung zweier langer Stents angedacht werden.

### Coronale Stents

- Wire Coil Design.
- Atraumatisches Einbringen ohne abrasive Effekte hier eine unerlässliche Bedingung und mit den Stents der vorliegenden Erfindung möglich.

### Design nicht-vaskularer Stents

Die wesentlichen Einsatzgebiete sind der gesamte Magen-Darm-Trakt, Luft- und Speiseröhre, Gallengang, Harnleiter, Harnröhre und Eileiter. Dementsprechend kommen Stents in den unterschiedlichsten Größen zum Einsatz. Die unterschiedlichen pH-Werte der Körperflüssigkeiten und das Auftreten von Keimen müssen im Stent-Design individuell berücksichtigt werden.

Unabhängig vom Einsatzort werden nicht-vaskulare Stents im Wesentlichen zur Drainage von Körperflüssigkeiten wie Gallensaft, Bauchspeicheldrüsensaft oder Urin eingesetzt. Daher empfiehlt sich ein Design eines perforierten Schlauchs, der zum einen die aus dem Hohlraum abzutransportierende Flüssigkeit sicher abführen kann, auf der anderen Seite jedoch über die komplette Strecke die Flüssigkeit aufnimmt. Weiterhin muss das verwendete Polymermaterial eine hohe Flexibilität aufweisen, um einen Tragekomfort sicherzustellen. Zur besseren Identifikation bei röntgenographischen Untersuchungen kann das Ausgangsmaterial mit Röntgenkontrastmitteln wie Bariumsulfat geblendet werden oder es werden rontgenopake Chromophore in die SMP-Materialien eingebaut, z.B. durch Einpolymerisieren geeigneter Monomere. Wenn Stents in Gebieten eingesetzt werden sollen, in denen Keime vorkommen, kann die Einarbeitung von antibiotischen Wirkstoffen in das Material sinnvoll sein.

Die insbesondere im uretheralen Bereich häufig auftretende Verkrustung der Stents kann durch geeignete Beschichtung oder Oberflächenmodifikation vermindert werden.

Die Fixierung des Stents hängt im Wesentlichen vom Einsatzort ab. Im Falle eines urethralen Stents findet sich das proximale Ende im Nierenbecken, das distale in der Harnblase oder auch außerhalb des Körpers. Hierbei formt das proximale Ende des Stents nach Abschluss der Expansion im Nierenbecken eine Schlaufe und sichert so den Halt.

Eine andere Möglichkeit zur Fixierung von Stents besteht darin, dass der Stent sich über radiale Kräfte nach außen hin fest an das umliegende Gewebe andrückt oder Anker-Elemente enthält, die der Fixierung dienen.

Im Falle von Gallen- oder Nierenstents ist eine atraumatische Platzierung und Entfernung eine unerlässliche Bedingung. Insbesondere muss hier bei der Platzierung sichergestellt sein, dass das Gewebe nicht durch abrasive Effekte in Mitleidenschaft gezogen wird und so Entzündungen hervorgerufen werden. Daher besitzt ein Stent, der in diesem Bereich eingesetzt wird, keinerlei zurückhaltende Elemente, die das Gewebe verletzen könnten.

Beispiele geeigneter Materialien, die im Rahmen der vorliegenden Erfindung eingesetzt werden können, sind im Folgenden exemplarisch dargelegt:

### PDL-PCL-Multiblockcopolymere

Das Multiblockcopolymer wurde aus Makrodiolen basierend auf Pentadecalacton (PDL) und ε-caprolacton (PCL) und einem Diisocyanat hergestellt. PDL gibt den Anteil an Pentadecalacton im Multiblockcopolymer an (ohne Berücksichtigung der Diisocyanatverbrückungen) sowie das Molgewicht der Polypentadecalactonsegmente. PCL gibt die entsprechenden Angaben für Caprolactoneinheiten an.

| Beispiel | PDL | PCL | Molgewicht Mₙ des Polyesterurethans | E-Modul (70°C / MPa) | Zugfestigkeit (MPa) |
|---|---|---|---|---|---|
| 1 | 100 Gew.-% / 10000 g/mol | | 192000 | 17 | 18 |
| 2 | 22 Gew.-% / 10000 g/mol | 78 Gew.-% / 10000 g/mol | 120000 | 1,4 | 5 |
| 3 | 41 Gew.-% / 10000 g/mol | 59 Gew.-% / 10000 g/mol | 196000 | 3 | 10 |
| 4 | 60 Gew.-% / 10000 g/mol | 40 Gew.-% / 10000 g/mol | 176000 | 7 | 8 |
| 5 | 80 Gew.-% / 10000 g/mol | 20 Gew.-% / 10000 g/mol | 185000 | 8,5. | 7 |
| 6 | 40 Gew.-% / 2000 g/mol | 60 Gew.-% / 4000 g/mol | 86000 | 3,5 | 4,5 |
| | | | | 35 (RT) | 23 (RT) |
| 7 | 50 Gew.-% / 3000 g/mol | 50 Gew.-% / 10000 g/mol | 75000 | 1,5 | 1,6 |
| | | | | 70 (RT) | 24 (RT) |
| 8 | 40 Gew.-% / 3000 g/mol | 60 Gew.-% / 10000 g/mol | 62000 | 3 | 9 |
| | | | | 45 (RT) | 30 (RT) |

Die mechanischen Eigenschaften in Abhängigkeit von der Temperatur für Beispiel 8 sind wie folgt:

| T | Bruchdehnung | E-Modul | Zugfestigkeit |
|---|---|---|---|
| (°C) | (%) | (MPa) | (MPa) |
| 22 | 900 | 45 | 30 |
| 37 | 1000 | 25 | 30 |
| 50 | 1000 | 12 | 20 |
| 55 | 1050 | 7 | 15 |
| 60 | 1050 | 3 | 10 |
| 65 | 1000 | 3 | 10 |
| 70 | 1000 | 3 | 9 |
| 75 | 1000 | 3 | 7 |
| 80 | 1000 | 1,5 | 3 |

## Patentansprüche

1. Stent zum Einsatz im nicht-vaskulären oder im vaskulären Bereich, umfassend ein bioabbaubares SMP-Material, wobei das SMP-Material ausgewählt ist unter kovalenten Polymernetzwerken und das SMP-Material ein Multiblock-Copolymer umfassend Poly(pentadecalacton)-Blöcke enthält.

2. Stent nach Anspruch 1, wobei der Stent ein Grundgerüst aus einem bioabbaubaren Kunststoff oder einem abbaubarem Metall aufweist, beschichtet mit dem SMP-Material.

3. Stent nach Anspruch 2, wobei das abbaubare Material eine Magnesiumlegierung, reines Magnesium oder ein Komposit aus Magnesium bzw. einer Magnesiumlegierung mit bioabbaubarem Polymer ist.

4. Stent nach einem der vorstehenden Ansprüche, weiter umfassend Additive, ausgewählt unter Röntgenkontrastmitteln und medizinisch wirksamen Verbindungen.

5. Stent nach einem der vorstehenden Ansprüche in der Form einer Röhre oder einer Röhre mit Perforation.

6. Stent nach Anspruch 1, wobei das Netzwerk ferner Poly(caprolacton)-Blöcke enthält.

7. Stent nach einem der vorstehenden Ansprüche, wobei der Stent zusätzlich eine Oberflächenbeschichtung aufweist.

8. Stent nach Anspruch 7, wobei die Oberflächenbeschichtung ausgewählt ist unter Beschichtungen, die die Hämokompatibilität modifizieren.

9. Stent nach einem der vorstehenden Ansprüche, ausgewählt unter Iliac-Stents, Renal-Stents, Karotis-Stents, Femoral-poplietal-Stents und Coronal-Stents.

10. Verfahren zur Herstellung eines Stents nach einem der vorstehenden Ansprüche, umfassend die Verarbeitung des SMP-Materials zu einem Stent durch Extrusionsverfahren, Beschichtungsverfahren, Formgussverfahren oder Spin- und Webverfahren.

11. Kit, umfassend einen Stent nach mindestens einem der Ansprüche 1 bis 9 und zusätzlich einen temperierbaren Ballonkatheter und/oder einen Ballonkatheter mit einem Lichtleiter.

## Claims

1. Stent for insertion in a non-vascular or vascular area, comprising a biodegradable SMP material, wherein the SMP material is selected among covalent polymer networks, and the SMP material contains a multiblock copolymer comprising poly(pentadecalactone) blocks.

2. The stent according to claim 1, wherein the stent features a basic structure of a biodegradable plastic or degradable metal, coated with the SMP material.

3. The stent according to claim 2, wherein the degradable material is a magnesium alloy, pure magnesium or a composite of magnesium or a magnesium alloy with biodegradable polymer.

4. The stent according to any one of the preceding claims, further comprising additives selected among X-ray contrast means and medically effective compounds.

5. The stent according to any one of the preceding claims in the form of a tube or a tube with perforation.

6. The stent according to claim 1, wherein the network further contains poly(caprolactone) blocks.

7. The stent according to any one of the preceding claims, wherein the stent additionally features a surface coating.

8. The stent according to claim 7, wherein the surface coating is selected among coatings which modify hemocompatibility.

9. The stent according to any one of the preceding claims, selected among lilac stents, renal stents, carotis stents, femoral-popliteal stents and coronal stents.

10. Method for producing a stent according to any one of the preceding claims, comprising the processing of the SMP material to create a stent through an extrusion method, coating method, mould-casting method or spinning and weaving method.

11. Kit comprising a stent according to at least one of claims 1 to 9 and additionally a balloon catheter, the temperature of which can be controlled, and/or a balloon catheter with an optical fibre.

## Revendications

1. Endoprothèse pour l'utilisation dans le domaine non vasculaire ou vasculaire, comprenant un matériau polymère à mémoire de forme biodégradable, le matériau polymère à mémoire de forme étant sélectionné parmi des réseaux polymères covalents et le matériau polymère à mémoire de forme contenant un copolymère multiblocs comprenant des blocs poly(pentadécalactone).

2. Endoprothèse selon la revendication 1, l'endoprothèse présentant une structure de base en une matière plastique biodégradable ou en un métal dégradable, revêtue avec le matériau polymère à mémoire de forme.

3. Endoprothèse selon la revendication 2, le matériau dégradable étant un alliage de magnésium, du magnésium pur ou un composé de magnésium ou d'un alliage de magnésium avec polymère biodégradable.

4. Endoprothèse selon l'une des revendications précédentes, comprenant en outre des additifs, sélectionnés parmi des agents de contraste radiologique et des composés médicalement efficaces.

5. Endoprothèse selon l'une des revendications précédentes sous la forme d'un tube ou d'un tube avec perforation.

6. Endoprothèse selon la revendication 1, le réseau contenant en outre des blocs poly(caprolactone).

7. Endoprothèse selon l'une des revendications précédentes, l'endoprothèse présentant en outre un revêtement de surface.

8. Endoprothèse selon la revendication 7, le revêtement de surface étant sélectionné parmi des revêtements modifiant l'hémocompatibilité.

9. Endoprothèse selon l'une des revendications précédentes, sélectionnée parmi des endoprothèses iliaques, des endoprothèses rénales, des endoprothèses carotidiennes, des endoprothèses fémoro-poplitées et des endoprothèses coronaires.

10. Procédé pour la fabrication d'une endoprothèse selon l'une des revendications précédentes, comprenant la transformation du matériau polymère à mémoire de forme en une endoprothèse par procédé d'extrusion, procédé de revêtement, procédé de moulage ou procédé de filage et tissage.

11. Ensemble comprenant une endoprothèse selon au moins une des revendications 1 à 9 et, en outre, un cathéter à ballonnet à température réglable et/ou un cathéter à ballonnet avec un guide de lumière.
